(19) Europäisches Patentamt
European Patent Office
Office européen des brevets

(11) Numéro de publication : **0 330 793 B1**

(12) **FASCICULE DE BREVET EUROPEEN**

(45) Date de publication du fascicule du brevet :
03.04.91 Bulletin 91/14

(51) Int. Cl.⁵ : **A61F 13/15**

(21) Numéro de dépôt : 88440057.3

(22) Date de dépôt : 04.07.88

(54) Couche-culotte.

(30) Priorité : 04.03.88 FR 8803142

(43) Date de publication de la demande :
06.09.89 Bulletin 89/36

(45) Mention de la délivrance du brevet :
03.04.91 Bulletin 91/14

(84) Etats contractants désignés :
AT BE CH DE ES GB GR IT LI LU NL SE

(56) Documents cités :
EP-A- 0 182 692
DE-A- 2 049 493
DE-A- 2 707 350
GB-A- 2 151 460
US-A- 3 840 013
US-A- 3 901 237
US-A- 3 971 380

(73) Titulaire : CELATOSE Société anonyme dite:
47, Rue de Bradford
F-59200 Tourcoing (Nord) (FR)

(72) Inventeur : Bruneau, Jean-Pierre
30 rue de la Visterie
Nomain F-59310 Orchies(Nord) (FR)

(74) Mandataire : Lepage, Jean-Pierre
Cabinet Lepage & Aubertin Innovations et
Prestations S.A. 23/25, rue Nicolas Leblanc
B.P. 1069
F-59011 Lille Cédex 1 (Nord) (FR)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 330 793 B1

Jouve, 18, rue Saint-Denis, 75001 PARIS

## Description

L'invention est relative à une couche culotte qui trouvera notamment son application dans le domaine de l'hygiène infantile et/ou médicale.

On connaît depuis de nombreuses années des couches culottes jetables. Leur aspect pratique et l'absence d'entretien ont largement contribué au développement de celles-ci qui ont fait l'objet de nombreux perfectionnements.

On connaît de telles couches culottes formées d'une feuille extérieure étanche, généralement d'un film en polyéthylène, et d'une feuille intérieure perméable de confort, généralement en voile de non-tissé, entre lesquelles est disposé un matelas formé de matériau absorbant.

De récents perfectionnements ont permis d'équiper de telles changes au niveau de l'entrejambe d'élastiques voire même au niveau de la ceinture afin d'augmenter l'étanchéité et le confort du porteur.

D'autres perfectionnements ont porté sur les systèmes de fermeture de la couche culotte et on connaît différentes réalisations qui, pour certaines d'entre elles, permettent le décollage et le recollage ultérieur du système adhésif sans détériorer la feuille de polyéthylène afin de pouvoir réutiliser la couche après toute intervention nécessitant le délangeage.

En effet, on connaît de nombreuses réalisations de couches culottes équipées d'au moins deux dispositifs d'attaches rapportés sur la feuille extérieure étanche au niveau de la ceinture à la partie arrière de la couche.

Certains perfectionnements de ces dispositifs d'attaches rapportés permettent la réutilisation de la couche même après plusieurs décollages et recollages successifs. A ce sujet, certains adhésifs ont été mis au point spécialement et on a trouvé certains compromis pour éviter l'arrachement du film extérieur lors du désassemblage de la couche.

Cependant, si de tels dispositifs d'attaches rapportés sur la couche ainsi perfectionnée présentent de nombreux avantages au niveau de l'utilisateur, il n'en est pas moins vrai que ceux-ci posent de nombreux problèmes aux producteurs de couches ; la phase de fabrication, dans laquelle les dispositifs d'attaches, sont rapportés étant très délicate à mettre en oeuvre.

En effet, pour fabriquer les couches généralement, on part d'une bande complexe en défilement continu et un dispositif mécanique très sophistiqué permet le découpage et la pose des systèmes de fermeture rapportés à partir d'une bobine de complexe adhésif en continu.

Un tel procédé de fabrication doit être surveillé et contrôlé en permanence pour vérifier le bon positionnement des dispositifs de fermeture rapportés ainsi que leur présence.

Cela étant, on connaît des documents EP-A-182.692, ou US-A-3.971.380, ou GB-A-2.151.460, ou encore US-A-3.901.237, une réalisation de couche culotte constituée d'une feuille extérieure étanche et d'une feuille intérieure perméable de confort entre lesquelles est disposé un matelas absorbant, les dites feuilles extérieure ou intérieure présentant plusieurs zones adhésivées coopérant directement avec les feuilles intérieure ou extérieure pour assurer la fermeture et le maintien de la couche.

De telles réalisations permettent d'éviter l'emploi de tout système de fermeture rapporté sur la couche tel que précisé ci-dessus mais nécessitent des dispositions ou une structure particulière pour protéger les zones adhésivées et éviter leur collage intempestif sur d'autres parties de la couche.

Le but de la présente invention est de proposer une couche culotte qui trouvera notamment son application dans le domaine de l'hygiène infantile et/ou médicale qui permette de pallier les inconvénients de la fabrication précitée notamment en évitant l'emploi de dispositifs traditionnels de fermeture rapportés sur la couche.

Un des buts de la présente invention est de proposer une couche culotte qui trouvera notamment son application dans le domaine de l'hygiène infantile et/ou médicale, qui bien que ne disposant pas de dispositifs traditionnels de fermeture rapportés sur la couche, autorise un maintien convenable de la couche au niveau de la ceinture sur l'individu.

Un autre but de la présente invention est de proposer une couche culotte, qui trouvera notamment son application dans le domaine de l'hygiène infantile et/ou médicale, équipée d'un système adhésif spécifique qui autorise des décollages et recollages successifs en donnant toute satisfaction.

Un autre but de la présente invention est de proposer une couche culotte dont le procédé de fabrication est simplifié et qui nécessite une installation moins sophistiquée, ce qui se répercutera sur le prix de revient final de la couche.

D'autres buts et avantages de la présente invention apparaîtront au cours de la description n'est cependant donnée qu'à titre indicatif et qui n'a pas pour but de la limiter.

Selon l'invention, la couche culotte, qui trouvera notamment son application dans le domaine de l'hygiène infantile et/ou médicale, formée d'une partie avant et d'une partie arrière réunies par une partie centrale constituant l'entrejambe, la dite couche présentant au moins :

— une feuille extérieure imperméable, au moins étanche au liquide,

— une feuille intérieure perméable de confort,

— un matelas de matériau absorbant, disposé entre les dites deux feuilles extérieure et intérieure,

— des moyens de fermeture, constitués par des zones de fixation adhésive prévues sur la cou-

2

che, aptes à réunir les parties avant et arrière de la couche pendant son utilisation pour assurer la fermeture et le maintien de la dite couche, est caractérisée par le fait que :

- les dits moyens de fermeture se présentent sous la forme d'au moins deux zones adhésivées disposées au niveau de la partie avant et arrière de la couche sur la dit feuille imperméable extérieure et/ou la dite feuille perméable intérieure, telles qu'elles soient aptes à être mises en vis-à-vis, pendant l'utilisation de la couche,

- les dites zones adhésivées étant non protégées, préalablement à l'utilisation de la couche, et aptes à coopérer ensemble lorsqu'elles sont placées en vis-à-vis.

La présente invention sera mieux comprise à la lecture de la description suivante accompagnée de dessins en annexe qui en font partie intégrante.

Les figures 1a et 1b illustrent une couche culotte selon un premier mode de réalisation de la présente invention, montrant respectivement la face interne et la face externe de la couche avant son utilisation.

Les figures 2a et 2b illustrent une couche culotte selon un second mode de réalisation de la présente invention montrant respectivement la face interne et la face externe de la couche avant son utilisation.

Les figures 3a et 3b illustrent une couche culotte selon un autre mode de réalisation de la présente invention montrant respectivement la face interne et la face externe de la couche avant son utilisation.

La figure 4 montre une vue en perspective d'une couche culotte, telle que représentée par exemple aux figures 1, formée pour son utilisation.

La figure 5 montre une vue en perspective d'une couche culotte, telle que représentée par exemple aux figures 2, formée pour son utilisation.

La figure 6 montre une vue en perspective d'une couche culotte, telle que représentée par exemple aux figures 3, formée pour son utilisation.

L'invention vise une couche culotte, qui trouvera notamment son application dans le domaine de l'hygiène infantile et/ou médicales.

Les couches culottes, et notamment les couches culottes dites jetables, subissent constamment une évolution et sont perfectionnées pour d'une part abaisser leur coût de revient et d'autre part pour faciliter leur utilisation.

L'objet de la présente demande porte sur des perfectionnements apportés au système de fermeture, et la présente invention se propose d'apporter une solution qui dispense des dispositifs de fermeture rapportés sur la couche.

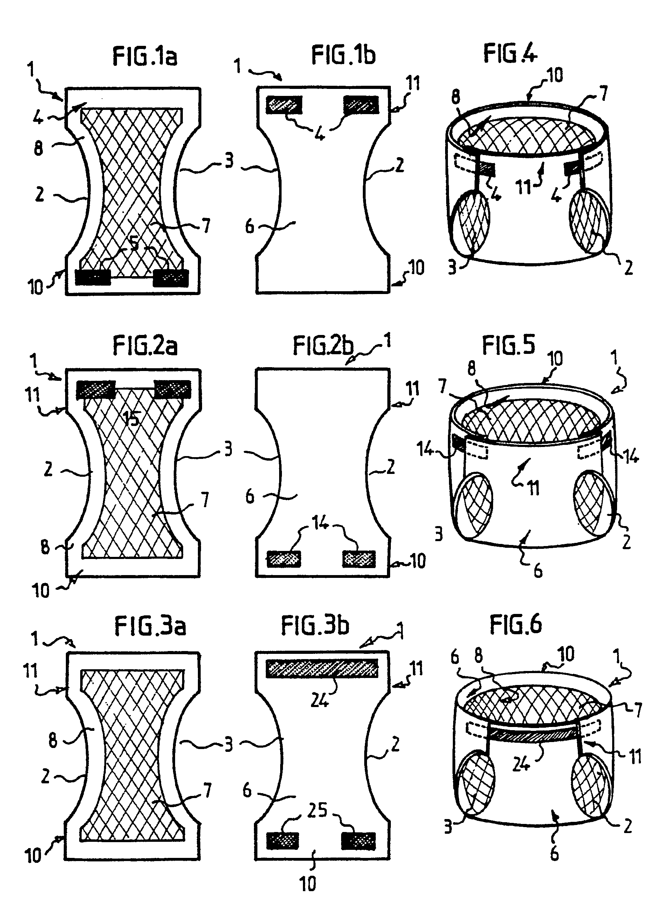

Les figures 1, 2 et 3 illustrent un modèle de couche culotte 1 de type traditionnel qui comporte des découpes latérales 2 et 3 pour permettre le passage des jambes. Il est à noter que de tels types de couches peuvent être équipés d'autres perfectionnements qui sortent de la présente invention, tels que par exemple présence d'élastiques au niveau des entrejambes ou autres.

En outre, pour ce qui est de la structure proprement dite de la couche culotte 1, elle est également formée traditionnellement par une feuille extérieure 6 étanche au moins aux liquides, généralement réalisée par un film de polyéthylène, ainsi qu'une feuille intérieure perméable de confort destinée à s'appliquer vers l'utilisateur, généralement réalisée en voile de non-tissé.

Entre la feuille extérieure étanche 6 et la feuille intérieure perméable 8 est disposé un matelas central de matériau absorbant 7, généralement constitué d'ouate de cellulose ou tout autre matériau absorbant voire même hyperabsorbant. A cet égard, pour former une enveloppe dans laquelle est renfermé le dit matériau absorbant 7, les feuilles extérieure 6 et intérieure 8 sont fixées mutuellement sur la périphérie de la couche culotte 1.

Ainsi formée, la couche culotte montre une partie centrale présentant les découpes 2 et 3 pour l'entrejambe réunissant d'une part une partie arrière 10 et d'autre part une partie avant 11.

Tout l'intérêt de la présente invention réside dans son système de fermeture de la couche au niveau de la ceinture qui sera formée sur l'individu par la réunion des parties arrière 10 et avant 11.

En effet, selon la présente invention, les dites feuilles extérieure 6 et/ou intérieure 8 présentent une ou plusieurs zones adhésivées 4, 5 ; 14, 15 ; 24, 25 au niveau de leur partie avant 11 et/ou arrière 10.

De plus, ces dites zones adhésivées coopèrent ensemble, lorsqu'elles sont placées en vis-à-vis, pendant l'utilisation de la couche, pour assurer la fermeture et le maintien de la couche culotte 1.

Ainsi, il est à remarquer qu'il n'est pas nécessaire d'employer des dispositifs traditionnels de fermeture rapportés sur la couche de part et d'autre de la partie arrière de la couche tels que jusqu'à présent.

Les figures 1, 2 et 3 illustrent des modes de réalisation utilisant pour la construction des zones adhésivées deux substances adhésivées complémentaires aptes à coopérer entre elles lorsqu'elles sont en vis-à-vis quand la couche culotte est positionnée.

Ainsi, les feuilles extérieures 6 et/ou intérieure 8 sont enduites selon le cas sur leur partie avant 11 et leur partie arrière 10 des dites substances adhésives complémentaires. Différents cas de figures non limitatifs sont imaginées aux figures 1, 2 et 3.

Par exemple, la figure 1 montre un premier mode de réalisation de couche selon l'invention dans lequel la feuille extérieure étanche 6 porte une ou plusieurs zones adhésives 4 sur la partie avant 11 et la feuille intérieure perméable 8 porte une ou plusieurs zones adhésivées 5 à la partie arrière 10 de la couche.

Les figures 2 montrent un second mode de réalisation, qui en quelque sorte est symétrique à celui de la figure 1 ; dans ce cas, la feuille extérieure 6 porte une ou plusieurs zones adhésivés 14 sur la partie arrière 10 alors que la feuille intérieure 8 porte une ou plusieurs zones adhésivées 15 sur la partie avant 11 de la couche.

La figure 3 montre un autre mode de réalisation dans laquelle la feuille extérieure 6 présente une ou plusieurs zones adhésivées 24 sur la partie avant 11 de la couche et également une ou plusieurs zones adhésivées 25 sur la partie arrière 10.

Les dites zones adhésivées se présentent sous la forme de pavés de colle disposés de part et d'autre des zones latérales des parties avant 11 et/ou arrière 10 de la couche 1. C'est le cas notamment des pavés repérés 4, 5, 14, 15, 25 aux figures 1 à 3.

Néanmoins, les dites zones adhésivées pourraient également se présenter sous la forme d'une bande de colle disposée transversalement au niveau des parties avant 11 et/ou arrière 10 de la couche 1. Un tel type de réalisation est par exemple illustré à la figure 3, zone repérée 21

Bien entendu, sur les figures ci-dessus mentionnées, les pavés formant les zones adhésivées sont schématisés et on pourrait imaginer d'autres formes que celles représentées.

Dans le cas d'utilisation de substances adhésives complémentaires comme il a été spécifié ci-dessus, pour faciliter la compréhension des dessins dans les modes de réalisation représentés aux figures 1, 2 et 3, le premier type de colle est respectivement repéré par 4, 14 et 24, alors que le second type de l'adhésif complémentaire est quant à lui repéré par 5, 15 et 25.

Naturellement, d'autres situations auraient pu être également envisagées sans pour autant sortir de la présente invention. Par exemple, d'une manière tout à fait symétrique à la réalisation représentée à la figure 3, on aurait pu envisager de positionner les pavés de colle 24, 25 sur la feuille intérieure 8 au niveau de l'avant 11 et de l'arrière 10, le repliage étant alors effectué symétriquement.

Dans la description précédente, on a évoqué le cas des substances adhésives complémentaires aptes à coopérer entre elles lorsqu'elles se retrouvent en vis-à-vis lorsque la couche est en position utilisation. Les figures 4, 5 et 6 montrent la fermeture de telles couches respectivement en utilisant les couches représentées aux figures 1, 2, et 3.

Dans le cas de la figure 4, la partie avant extérieure de la couche 6-11, portant les pavés de colle 4, est ensuite recouverte par les deux parties latérales arrière intérieures 10-8, portant elles les pavés complémentaires 5.

Dans la figure 5, on est en présence de la situation inverse, c'est-à-dire que les pavés adhésifs 11 présents sur la partie arrière extérieure 10-6 sont eux recouverts par les pavés 15 portés par la partie avant intérieure 11-8.

Quant à la figure 6, les pavés 24, 25 complémentaires sont portés par la même feuille, à savoir la feuille étanche 6, et pour autoriser la fermeture de la couche, on replie avantageusement la feuille étanche 6 vers la feuille perméable 8, au niveau de la partie arrière 10, de façon à ce que les pavés 25 de la dite partie arrière extérieure 10-6 soient en vis-à-vis au moment du positionnement de la couche du ou des pavés complémentaires 24, portés par la feuille étanche 6 au niveau de la partie avant 11.

En outre, il est à remarquer que la feuille intérieure 8, qui généralement est constituée en voile de non-tissé, pourra présenter au niveau des zones adhésivées 5-15 un état de surface renforcé pour assurer une bonne tenue de l'adhésif et un bon maintien de la couche fermée.

Contrairement aux couches culottes connues qui utilisent un adhésif sensible à la pression, coopérant directement avec la feuille extérieure ou intérieure correspondant en vis-à-vis lorsque la couche est placée en position sur l'individu, et rendant indispensable par exemple l'utilisation de feuilles protectrices pelables des zones adhésives, pour éviter que cette substance adhésive n'adhère intempestivement aux autres parties de la couche, selon la présente invention, cette protection n'est pas nécessaire.

En effet, selon la présente invention, on pourra se passer de telles feuilles protectrices en utilisant des adhésifs complémentaires tels qu'ils aient chacun un faible pouvoir adhésif, voire même nul, et que seule la présence de l'autre matière adhésive complémentaire permette le collage.

En outre, on utilisera avantageusement des colles adhésives complémentaires qui permettent le repositionnement c'est-à-dire qui autorisent un décollage et des recollages successifs afin d'autoriser un éventuel délangeage sans pour autant rendre la couche inutilisable après ouverture.

En ce qui concerne la structure des matières adhésives complementaires, on pourra utiliser des colles connues de l'homme de l'art.

On pourrait égalaient envisager d'utiliser des colles ou matières adhésives incluses dans des microcapsules éclatant sous l'effet de la pression.

Par ailleurs, au lieu d'enduire les dites feuilles des dites substances adhésives, on pourrait également envisager l'emploi d'éléments adhésifs double face rapportés sur les dites feuilles.

## Revendications

1. Couche culotte (1), qui trouvera notamment son application dans le domaine de l'hygiène infantile et/ou médicale, formée d'une partie avant (11) et d'une partie arrière (10) réunies par une partie centrale constituant l'entrejambe (2, 3), la dite couche présentant au moins :

– une feuille extérieure imperméable (6), au moins étanche au liquide,

– une feuille intérieure perméable (8) de confort,

– un matelas de matériau absorbant (7), disposé entre les dites deux feuilles extérieure (6) et intérieure (8),

– des moyens de fermeture, constitués par des zones de fixation adhésive prévues sur la couche (1), aptes à réunir les parties avant (11) et arrière (10) de la couche (1) pendant son utilisation pour assurer la fermeture et le maintien de la dite couche (1), caractérisée par le fait que :

– les dits moyens de fermeture se présentent sous la forme d'au moins deux zones adhésivées (4, 5 ; 15, 14 ; 24, 25) disposées au niveau de la partie avant (11) et arrière (10) de la couche (1) sur la dite feuille inperméable extérieure (6) et/ou la dite feuille perméable intérieure (8), telles qu'elles soient aptes à être mises en vis-à-vis, pendant l'utilisation de la couche,

– les dites zones adhésivées étant non protégées, préalablement à l'utilisation de la couche, et aptes à coopérer ensemle lorsqu'elles sont placées en vis-à-vis.

2. Couche culotte selon la revendication 1, caractérisée par le fait que les dites zones (4, 5 ; 15, 14 ; 24, 25) adhésivées sur la partie avant (11) et arrière (10) de la couche (10) sont constituées au moyen de deux substances adhésives complémentaires aptes à coopérer entre elles lorsqu'elles sont en vis-à-vis quand la couche culotte est positionnée.

3. Couche culotte selon la revendication 1, caractérisée par le fait que les dites zones adhésivées (4, 5 ; 15, 14 ; 24, 25) se présentent sous la forme de pavés de colle, disposés de part et d'autre dans les zones latérales des parties avant (11) et/ou arrière (10) de la couche (1).

4. Couche culotte selon la revendication 1, caractérisée par le fait que les dites zones adhésivées (4, 5 ; 15, 14 ; 24, 25) se présentent sous la forme d'une bande enduite, disposée transversalement au niveau des parties avant (11) et/ou arrière (10) de la couche (1).

5. Couche culotte selon la revendication 2, caractérisée par le fait que la dite feuille extérieure (6) présente une ou plusieurs zones adhésivées (4) sur la partie avant (11) de la couche et que la dite feuille (8) présente une ou plusieurs zones adhésivées (5) sur la partie arrière (10) de la couche (1).

6. Couche culotte selon la revendication 2, caractérisée par le fait que la dite feuille extérieure (6) présente une ou plusieurs zones adhésivées (14) sur la partie arrière (10) de la couche et que la dite feuille intérieure (8) présente une ou plusieurs feuilles adhésivées (15) sur la partie avant (11) de la couche (1).

7. Couche culotte selon la revendication 2, caractérisée par le fait que la dite feuille extérieure (6) présente une ou plusieurs zones adhésivées (24, 25) sur la partie arrière (10) et avant (11) de la couche.

8. Couche culotte selon la revendication 2, caractérisée par le fait que la dite feuille intérieure (8) présente une ou plusieurs zones adhésivées sur la partie arrière (10) et avant (11) de la couche.

## Ansprüche

1. Hosewindel (1), die ihre Anwendung nämlich auf dem Gebiet der Rinder- und/oder medizinischen Hygiene finden wird, bestehend aus einem Vorderteil (11) und einem Hinterteil (10), die von einem Mittelteil, der die Beinenöffnungen (2, 3) bildet, wobei die genannte Windel zumindest aufweist :

– eine undurchlässige, zumindest flüssigkeitendichte Außenfolie (6),

– eine durchlässige Komfortinnenfolie (8),

– eine Polsterschicht aus einsaugendem Material (7), die zwischen den beiden genannten Außen- (6) und Innenfolien (8) angeordnet ist,

– Verschlußmittel, bestehend aus Klebbefestigungsbereichen auf der Windel (1), die geeignet sind, den Vorderteil (11) mit dem Hinterteil (10) der Windel (1) während deren Verwendung zu vereinigen, um den Verschluß und die Haltung der genannten Windel (1) zu sichern,

dadurch gekennzeichnet, daß :

– die gennanten Verschlußmittel als zumindest zwei klebfähige Bereiche (4, 5 ; 15, 14 ; 24, 25) ausgebildet sind, die im Bereich des Vorderteils (11) und des Hinterteils (10) der Windel (1) auf der genannten undurchlässigen Außenfolie (6) und/oder auf der genannten durchlässigen Innenfolie so angeordnet sind, daß sie geeignet sind, während der Verwendung der Windel gegenüber einander gebracht zu werden,

– die genannten klebfähigen Bereiche vor der Verwendung der Windel ungeschützt und geeignet sind, zusammenzuwirken, wenn sie gegenüber einander gebracht werden.

2. Hosewindel nach Anspruch 1, dadurch gekennzeichnet, daß die genannten klebfähigen Bereiche (4, 5 ; 15, 14 ; 24, 25) am Vorderteil (11) und am Hinterteil (10) der Windel (1) aus zwei einander ergänzenden Klebsubstanzen bestehen, die geeignet sind, mit einander zusammenzuwirken, wenn die Hosewindel positioniert ist.

3. Hosewindel nach Anspruch 1, dadurch gekennzeichnet, daß die genannten klebfähigen Bereiche (4, 5 ; 15, 14 ; 24, 25) als an beiden Seiten in den Seitenbereichen des Vorderteils (11) und/oder des Hinterteils (10) der Windel (1) angeordnete Klebepflaster ausgebildet sind.

4. Hosewindel nach Anspruch 1, dadurch gekennzeichnet, daß die genannten klebfähigen Bereiche (4, 5 ; 15, 14 ; 24, 25) als ein im Bereich des Vorderteils (11) und/oder des Hinterteils (10) der Windel (1) quer angeordneter, verleimter Streifen ausgebildet sind.

5. Hosewindel nach Anspruch 2, dadurch gekennzeichnet, daß die genannte Außenfolie (6) einen oder mehrere klebfähige Bereiche (4) am Vorderteil (11) der Windel und die genannte Innenfolie (8) einen oder mehrere klebfähige Bereiche (5) am Hinterteil (10) der Windel (1) aufweisen.

6. Hosewindel nach Anspruch 2, dadurch gekennzeichnet, daß die genannte Außenfolie (6) einen oder mehrere klebfähige Bereiche (14) am Hinterteil (10) der Windel und die genannte Innenfolie (8) einen oder mehrere klebfähige Bereiche (15) am Vorderteil (11) der Windel (1) aufweisen.

7. Hosewindel nach Anspruch 2, dadurch gekennzeichnet, daß die genannte Außenfolie (6) einen oder mehrere klebfähige Bereiche (24, 25) am Hinterteil (10) und am Vorderteil (11) der Windel aufweist.

8. Hosewindel nach Anspruch 2, dadurch gekennzeichnet, daß die genannte Innenfolie (8) einen oder mehrere klebfähige Bereiche am Hinterteil (10) und am Vorderteil (11) der Windel aufweist.

## Claims

1. Diaper pants (1), which are particularly applicable in the field of child and/or medical hygiene, formed by a front portion (11) and a rear portion (10) joined by a central portion constituting the crutch (2, 3), the said diaper having at least :
   - an impermeable external sheet (6), at least liquid tight,
   - a permeable, comfort ensuring internal sheet (8),
   - a padding of absorbent material (7), placed between the said two external (6) and internal (8) sheets,
   - closing means, constituted by adhesive fixing areas provided on the diaper (1), suitable for joining the front (11) and rear (10) portions of the diaper (1) during its use to ensure that the said diaper (1) is closed and held in place, characterized by the fact that :
   - the said closing means take the form of at least two areas rendered adhesive (4, 5 ; 15, 14 ; 24, 25) arranged at the front (11) and rear (10) portions of the diaper (1) on the said external impermeable sheet (6) and/or the said internal permeable sheet (8), such that they are suitable for being placed opposite one another while the diaper is being used,
   - the said areas rendered adhesive being unprotected, prior to use of the diaper, and suitable for cooperating with one another when they are placed opposite.

2. Diaper pants according to claim 1, characterized by the fact that the said areas rendered adhesive (4, 5 ; 15, 14 ; 24, 25) on the front (11) and rear (10) portions of the diaper (1) are constituted by means of two complementary adhesive substances suitable for cooperating with one another when they are opposite when the diaper pants are positioned.

3. Diaper pants according to claim 1, characterized by the fact that the said areas rendered adhesive (4, 5 ; 15, 14 ; 24, 25) take the form of blocks of glue, disposed on either side in the lateral areas of the front (11) and/or rear (10) portions of the diaper (1).

4. Diaper pants according to claim 1, characterized by the fact that the said areas rendered adhesive (4, 5 ; 15, 14 ; 24, 25) take the form of a coated strip, arranged transversely at the front (11) and/or rear (10) portions of the diaper (1).

5. Diaper pants according to claim 2, characterized by the fact that the said external sheet (6) has one or more areas rendered adhesive (4) on the front portion (11) of the diaper and that the said sheet 18) has one or more areas rendered adhesive (5) on the rear portion (10) of the diaper (1).

6. Diaper pants according to claim 2, characterized by the fact that the said external sheet (6) has one or more areas rendered adhesive 114) on the rear portion (10) of the diaper and that the said internal sheet (8) has one or more sheets rendered adhesive (15) on the front portion (11) of the diaper (1).

7. Diaper pants according to claim 2, characterized by the fact that the said external sheet (6) has one or more areas rendered adhesive (24, 25) on the rear (10) and front (11) portions of the diaper.

8. Diaper pants according to claim 2, characterized by the fact that the said internal sheet (8) has one or more areas rendered adhesive on the rear (10) and front (11) portions of the diaper.

FIG.1a

FIG.1b

FIG.4

FIG.2a

FIG.2b

FIG.5

FIG.3a

FIG.3b

FIG.6